| | Europäisches Patentamt | |
|---|---|---|
| (19) | European Patent Office | |
| | Office européen des brevets | (11) **EP 0 871 509 B1** |

(12) **EUROPEAN PATENT SPECIFICATION**

(45) Date of publication and mention
 of the grant of the patent:
 **30.08.2006 Bulletin 2006/35**

(21) Application number: **96941560.3**

(22) Date of filing: **20.12.1996**

(51) Int Cl.:
 **A61M 16/00** *(2006.01)*    **A61B 5/087** *(2006.01)*

(86) International application number:
 **PCT/CA1996/000866**

(87) International publication number:
 **WO 1997/022377 (26.06.1997 Gazette 1997/27)**

(54) **DEVICE FOR THE DETERMINATION OF AT LEAST TWO PARAMETERS OF A PATIENT'S RESPIRATORY SYSTEM AND METHOD THEREFOR**

EINRICHTUNG ZUR MESSUNG VON MINDESTENS ZWEI PNEUMATISCHEN LUNGPARAMETERN UND MESSVERFAHREN DAZU

DISPOSITIF DE DETERMINATION D'AU MOINS DEUX PARAMETRES RELATIFS AU SYSTEME RESPIRATOIRE D'UN PATIENT ET PROCEDE ASSOCIE

(84) Designated Contracting States:
 **AT BE CH DE DK ES FI FR GB GR IE IT LI LU MC NL PT SE**

(30) Priority: **20.12.1995 GB 9525994**

(43) Date of publication of application:
 **21.10.1998 Bulletin 1998/43**

(73) Proprietor: **The University of Manitoba
 Winnipeg,
 Manitoba R3T 5V4 (CA)**

(72) Inventor: **YOUNES, Magdy
 Winnipeg, Manitoba R3M 0J2 (CA)**

(74) Representative: **Lawrence, Malcolm Graham et al
 HLBBshaw
 Merlin House
 Falconry Court
 Baker's Lane
 Epping, Essex CM16 5DQ (GB)**

(56) References cited:
 **WO-A-82/01654          WO-A-92/11054
 DE-A- 4 038 871          GB-A- 2 077 444
 US-A- 3 871 371          US-A- 5 107 830
 US-A- 5 318 038**

EP 0 871 509 B1

**Description**

**FIELD OF THE INVENTION**

The present invention relates to ventilation and, in particular, to assisted modes of ventilation.

**BACKGROUND TO THE INVENTION**

Determination of elastic and resistive properties of the respiratory system is of considerable importance in monitoring disease progression regardless of ventilator mode used. In the proportional assist mode of ventilation, knowledge of these properties is, in addition, essential for proper adjustment of the volume-related and flow-related assist gains. The proportional assist mode of ventilation (PAV®) is fully described in U.S. Patent No. 5,107,830 (Younes).
Briefly, in proportional assist ventilation, the pressure delivered by the ventilator increases in direct proportion to patient effort and the proportionality applies from breath to breath as well as continuously throughout each inspiration.

Proportional assist ventilation operates on the principle that the inspiratory flow $\cdot(\overset{\bullet}{V})$ and its integral, volume (V) of a patient contain the necessary information to substantially match the profile of patient effort. By continuously measuring the instantaneous values of flow and volume and applying a gain factor appropriate to each (for flow, cmH$_2$O/L/S and for volume cmH$_2$O/ml) , the ventilator can deliver a pressure profile to the patient that amplifies the instantaneous pressure generated in the patient.
The pressure assist provided to the patient may be expressed by the relationship:

$$P_{vent} = K_1V + K_2\overset{\bullet}{V}$$

where P$_{vent}$ is the magnitude of the pressure assist, K$_1$ is a gain factor applied to a variable ongoing volume signal (V) and K$_2$ is a gain factor applied to a variable ongoing flow signal $(\overset{\bullet}{V})$. The K$_1$ (or VA) and K$_2$ (or PA) values are fractions of respiratory elastance and respiratory resistance respectively.
In apneic patients, the ventilator provides the only distending force, as reflected by airway pressure (P$_{aw}$). Because P$_{aw}$ provides the total distending pressure, it is possible to reliably determine the elastic and resistive properties of such patients. A variety of reliable methods have been described in the literature for this purpose.
The situation is much more complex in the assist modes of ventilation where ventilator cycle is synchronized with patient's inspiratory effort. In this case, P$_{aw}$ is not the only distending force. Rather, flow $(\overset{\bullet}{V})$ and volume (V) are generated as a result of the combined action of the ventilator (as reflected by P$_{aw}$) and the patient (as expressed in muscle pressure (P$_{mus}$)). To the extent that the magnitude of P$_{mus}$ is continuously varying and cannot be measured or estimated without prior knowledge of patient mechanics, it is not possible to estimate total applied pressure (i.e. P$_{aw}$ + P$_{mus}$) at any instant of the inspiratory phase of the ventilator cycle. This problem has made it difficult to reliably estimate mechanical properties in the assist modes.
Given that the behaviours of P$_{aw}$, flow and volume during a "normal" cycle cannot be used to estimate mechanical properties, one is left with the option of causing a perturbation in one of the primary variables (e.g. P$_{aw}$ or $\overset{\bullet}{V}$) and observing the consequences on the other variables. This approach, which has been used successfully in the controlled ventilation modes, is fraught with difficulties in the assist modes, since the perturbation produced at the airway may alter the pressure generated by the patient (P$_{mus}$) via at least three mechanisms, namely:

(a) Mechanical perturbations are readily perceived. The patient may react at a behavioural level, altering P$_{mus}$;
b) The change in flow or volume produced by the perturbation may alter P$_{mus}$ reflexly (i.e. independent of perception); and
c) The change in flow or volume may alter P$_{mus}$ at a strictly mechanical level via the intrinsic properties of respiratory muscles (force-length and force-velocity relations).

If P$_{mus}$ is altered by the perturbation, then one cannot assume that the change in P$_{aw}$ during the perturbation represents the total change in applied force which, in the assist mode, is given by [ ▲ P$_{aw}$ + ▲P$_{mus}$]. This relationship,

again, makes it impossible to use the relation between $P_{aw}$, $\overset{\bullet}{V}$ and V during the perturbation to estimate patient mechanics, unless it can be assured that the perturbation does not change $P_{mus}$.

SUMMARY OF INVENTION

The methods described in detail below and provided in accordance with the present invention make it possible to circumvent the above difficulties, thereby permitting reliable estimates of passive elastic and resistive properties in the face of continuously varying, and unquantifiable, $P_{mus}$, during the assist mode of ventilation. The procedures employed to estimate the passive pressure-volume relationship and the passive pressure-flow relationship are separate procedures but may be combined, as described in detail below.

In one aspect of the present invention, as defined in claim 1, there is provided a method for estimating a passive pressure-volume relationship of a respiratory system in a patient on mechanical ventilatory support and producing spontaneous respiratory effort (assist mode of ventilation) to guide selection of a volume-assist component of the proportional assist ventilation mode (PAV) which comprises:

a) placing a ventilator in the proportional assist mode of ventilation, using empiric values of elastance and resistance or values of elastance and resistance determined by other conventional methods, for initial adjustment of the volume-related and flow-related assist components of the proportional assist ventilation,

b) monitoring airway pressure ($P_{aw}$) and flow (V) and volume (V) to the patient,

c) holding flow at or near zero in selected breaths for a period beyond termination of an inspiratory phase of the ventilation,

d) measuring $P_{aw}$ at a point as far away as possible from the onset of the inspiratory hold but sooner than the latency for behavioral respiratory responses to provide $P_{hold}$,

e) measuring the tidal volume ($V_T$) of the breaths selected for the inspiratory hold step,

f) establishing the relationship between $P_{hold}$ and $V_T$ in said selected breaths to provide a pressure-volume relationship over the $V_T$ range encountered during proportional assist ventilation to permit subsequent adjustment of the volume-related assist for proportional assist ventilation.

In another aspect of the present invention, as defined in claim 16, there is provided an apparatus for estimating a passive pressure-volume relationship of a respiratory system in a patient on mechanical ventilatory support and producing spontaneous respiratory effort (assist mode of ventilation) to guide selection of a volume-assist component of the proportional assist ventilation mode (PAV), which comprises:

a) means for placing a ventilator in the proportional assist mode of ventilation, using empiric values of elastance and resistance, or values of elastance and resistance determined by other conventional methods, for initial adjustment of the volume-related and flow-related assist components of the proportional assist ventilation,

b) means for monitoring airway pressure ($P_{aw}$) and flow (V) and volume (V) to the patient,

c) means for holding flow at or near zero in selected breaths for a period beyond termination of an inspiratory phase of the ventilation,

d) means for measuring $P_{aw}$ at a point as far away as possible from the onset of the inspiratory hold but sooner than the latency for behavioral respiratory responses to provide $P_{hold}$,

e) means for measuring the tidal volume ($V_T$) of the breaths selected for the inspiratory hold step,

f) means for establishing the relationship between $P_{hold}$ and $V_T$ in said selected breaths to provide a pressure-volume relationship over the $V_T$ range encountered during proportional assist ventilation to permit subsequent adjustment of the volume-related assist for proportional assist ventilation.

BRIEF DESCRIPTION OF DRAWINGS

Figure 1 illustrates the typical pattern of respiratory motor output during a normal respiratory cycle established through decades of investigation in respiratory control;

Figure 2, which comprises graphs A, B and C, contains graphs showing the airway pressure ($P_{aw}$) (Graph A), Volume (Graph B) and Flow (Graph C) during an inspiratory hold manoeuvre as practised herein in accordance with one embodiment of the invention;

Figure 3, which comprises graphs a, b, c, d, e and f, shows the graphical relationship of $P_{hold}$ to $V_T$ for a series of collections of observations made herein; and

Figure 4, which comprises graphs A and B, shows the plots of $P_{aw}$, Flow and Volume for positively perturbed (Graph A) and negatively perturbed (Graph B) breaths, in accordance with another embodiment of the invention.

## GENERAL DESCRIPTION OF INVENTION

The ability to establish passive elastic and resistance values for a particular patient as provided herein arises from several developments in the field of mechanical ventilation.

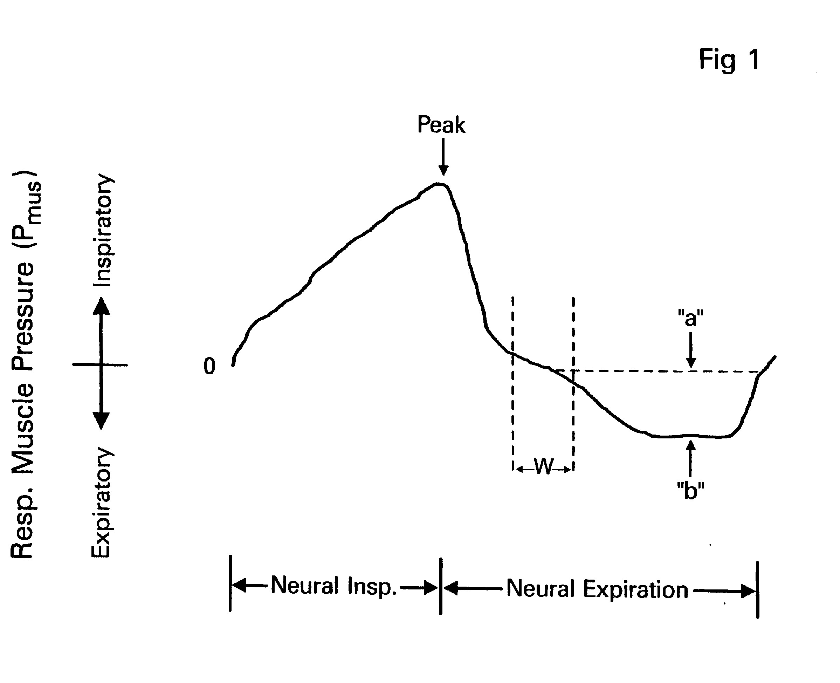

Figure 1 illustrates the typical pattern of respiratory motor output during a respiratory cycle as established through decades of investigation in respiratory control. There is a ramp increase in inspiratory muscle activity (or inspiratory $P_{mus}$). After reaching a peak level, the inspiratory output decreases, usually rapidly, towards zero. When the drive to breathe is low, $P_{mus}$ generally stays at zero until the beginning of the next cycle (dashed line "a", Figure 1).

With a high drive, or with delayed emptying during expiration, expiratory muscles may be recruited. When this happens, the pressure generated by expiratory muscles is minimal early in neural expiration and builds up to a maximum later in expiration (line "b", Figure 1).

There is, accordingly, a time window (w, Figure 1), during which $P_{mus}$ is near zero regardless of whether expiratory muscle activity is present or not. Because, in the vicinity of this window, the rate of change in inspiratory or expiratory $P_{mus}$ is relatively small, there is a relative insensitivity to errors in estimating where this window is. Given extensive personal experience of the inventor in monitoring $P_{mus}$ in various conditions, in ventilated patients, "w" should begin within about 200 to about 400 msec of peak inspiratory $P_{mus}$.

The inspiratory hold technique has been in use for many years to measure elastic recoil at end-inspiration both in controlled and assist modes of ventilation. Barring behavioural responses, in the controlled modes (apneic or paralysed patient), the pressure measured during this holding time (plateau pressure) reflects passive elastic recoil. In the assist modes, the plateau pressure is contaminated by an indeterminate amount related to $P_{mus}$, regardless of whether behavioural responses occur.

In the conventional assist modes (volume cycled [assist/control] and pressure cycled [PSV]), there is no linkage between the end of ventilator inspiratory cycle and the end of patient's neural cycle. The inflation phase may terminate at any time during the patient's cycle. The inspiratory hold period may thus coincide with a period during which there is a large inspiratory or expiratory $P_{mus}$.

By contrast, in the proportional assist mode of ventilation (PAV®), the end of the ventilator inflation phase is dictated to occur during the phase of rapid decline in inspiratory $P_{mus}$, very close to "w". If plateau pressure is measured within a few hundred msecs of end of inflation phase, the likelihood of there being significant inspiratory or expiratory $P_{mus}$ is reduced substantially. This phenomenom is employed herein in one aspect of the invention.

The inventor has found that the minimum latency to execute a behavioural response in $P_{mus}$ following a mechanical perturbation at the airway is at least about 200 msec. This is the case even in alert healthy subjects who are prewarned to react as fast as possible to an anticipated intervention. In mechanically ventilated patients, behavioural responses are rarely observed before about 300 msec of a perturbation, even when the patient is awake. This result is related to the usual obtundation and lack of anticipation. It follows that measurements in such patients made within about 300 msec of a mechanical perturbation at the airway are not likely to be subject to errors produced by behavioral responses leading to a change in $P_{mus}$.

The inventor has measured $P_{mus}$ in a large number of subjects in the course of brief (< about 200 msec) perturbations of $P_{aw}$ and flow. The inventor found that the changes in $P_{mus}$ produced by modest changes in $P_{aw}$ or flow (of the magnitude utilized herein) are too small to cause a significant error in estimated mechanical properties. Reflex and mechanical feedback altering $P_{mus}$ in relation to flow and volume are, therefore, not important during perturbations of the kind and magnitude utilized herein.

Although the procedures for estimating the passive elastic (pressure-volume relation) and resistive (pressure-flow relation) are described separately below, they in fact do share common features and requirements and are intended to operate concurrently using a common computer program to provide continuous information on these two properties which is needed for adjustment of the two essential components of PAV, the volume-related and flow-related assist gains.

## DESCRIPTION OF PREFERRED EMBODIMENTS

The procedure provided herein now will be described in detail with respect to a preferred embodiments thereof. The invention involves several aspects, as now set forth:

1) Procedure for Automatic Determination of the Passive Elastic Properties (Pressure-Volume Relationship) (first aspect of invention):

In current ventilation practice, elastance ($E_{rs}$) in the assist modes of ventilation is most commonly measured by applying an inspiratory hold, measuring the $P_{aw}$ when a reasonably stable $P_{aw}$ plateau is reached ($P_{plat}$), measuring the corresponding tidal volume ($V_T$) and applying the following equation:

$$E_{rs} = (P_{plat} - PEEP)/V_T$$

where PEEP is .the pressure at end-expiration of the ventilation cycle, which is usually above zero in mechanically ventilated patients. Apart from the fact that $P_{plat}$ may be contaminated by inspiratory or expiratory $P_{mus}$, and hence not reflecting passive elastic recoil, the current approach incorporates a serious potential error in that the approach assumes that elastic recoil pressure at the beginning of inspiration (i.e. $P_{EL}EE$) equals PEEP. This is clearly an untenable assumption, since end-expiratory volume (i.e. volume at beginning of inspiratory phase) may be higher than passive functional residual capacity (FRC), due to dynamic hyperinflation, resulting in $P_{EL}EE>PEEP$, or lower than passive FRC, due to expiratory muscle activity, thereby resulting in $P_{EL}EE<PEEP$. That these collective errors are serious, is evident from the wide variability in elastance measurements between consecutive determinations and the lack of any good agreement between elastance determination measured in the assist modes using the current approach and determinations made in the same patient after induced passivity and after establishing that the volume at the beginning of the inspiratory phase is, indeed, passive FRC.

In accordance with one embodiment of the invention, these problems are addressed to provide the pressure-volume relationship in the following way:

1) PAV is used as the assist mode of ventilation. In this way, the onset of the inspiratory hold is dictated to coincide with the rapid declining phase of inspiratory $P_{mus}$, close to the point where $P_{mus}$ is near zero.

2) The pressure during the hold is measured at a time less than the latency for behavioural responses, thereby eliminating behavioural responses as a source of contamination of $P_{plat}$, and hence as a source of errors in estimating passive elastance.

3) There is no assumption that elastic recoil at the end of expiration (beginning of inspiration) equals PEEP. Rather, the relationship between $V_T$ and $P_{plat}$, determined according to steps (1) and (2) above, and subsequently referred to as $P_{hold}$, is determined independent of PEEP, taking advantage of the normally wide $V_T$ variability during PAV or, in the absence of spontaneous variability, of deliberate procedures to obtain such a wide range. The intercept of the relation between $V_T$ and $P_{hold}$ may or may not equal PEEP and any differences that may exist provide additional information regarding the difference between end-expiratory volume and passive FRC, or non-linearities in the pressure-volume relation, both of which are very difficult to obtain otherwise, while being of critical importance in adjusting the volume-related assist in PAV.

The elastance determined according to this approach is different from what is referred to as static elastance, since $P_{aw}$ is measured well before the recommended about 2 to 5 sec of inspiratory hold for determination of true static elastance. $P_{hold}$, and hence elastance estimated by procedure of the invention, overestimates static elastic pressure, and hence static elastance, because viscoelastic pressure may not have completely dissipated at about 200 to 300 msec of the hold manoeuvre.

Although measurements made according to the present invention may not truly reflect static elastic behaviour, they are more appropriate for determining elastic behaviour during the inspiratory phase than any prior art procedure and are, hence, more relevant to adjusting the volume-assist gain of PAV.

Preferred Procedure for Pressure-Volume Relationship:

The patient first is placed in the PAV mode of ventilation. Provision is made for monitoring $P_{aw}$, flow and volume. Initial settings of flow assist (FA) and volume assist (VA) on the ventilator may be based on previously determined values of resistance (R) and elastance (E) in the controlled mode of ventilation. Alternatively, default values for FA and VA may be used. The primary intent of these values is to provide some assist but, at the same time, remain below the patient's resistance (R) and elastance (E), respectively. So long as this relationship is maintained, the end of ventilator inflation phase coincides with the phase of decline in inspiratory $P_{mus}$ (see Figure 1 and above discussion thereof). Suitable default values may be FA = [endotracheal (ET) tube resistance +4], and VA = 10 cmH$_2$O/l. If these initial settings are clearly inadequate for the patient as evident by distress, VA and/or FA may be increased gradually but must remain below values that cause a runaway condition, as described in the aforementioned U.S. Patent No. 5,107,830.

Beyond these initial settings, elastic properties may be determined according to the following paradigm and VA can subsequently be readjusted in light of its results.

The basic procedure is to implement an inspiratory hold manoeuvre and to measure $P_{aw}$ at a point within about 300 msec from the end of inflation phase. For the reasons discussed above, $P_{aw}$ can be measured beyond this point. However, the farther out one goes, the greater the likelihood of contamination with behavioral responses as well as $P_{exp}$ unrelated to behavioral responses.

To minimize anticipatory responses from the patient, the inspiratory hold manoeuvre preferably should not be applied to every breath but rather in random sequence at an average frequency that would allow there to be obtained a reasonable number of data points (e.g. about 15 to 20) in a reasonable time (e.g. about 5 to 20 min). The frequency of such application may also be decreased when results are fairly stable.

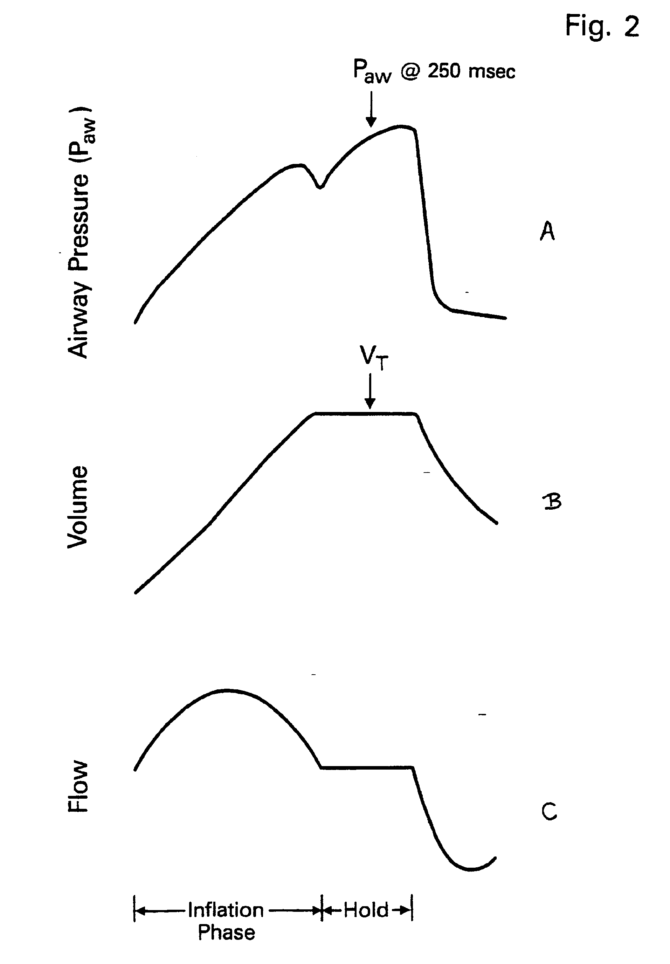

For each inspiratory hold manoeuvre, $P_{aw}$ at about 250 msec from the onset of the inspiratory hold (i.e. zero flow) and the corresponding tidal volume ($V_T$) are noted (see Figure 2). These data are stored. Ideally, the pressure-volume relation should be defined over a wide $V_T$ range. Patients in the PAV ventilation mode usually display substantial breath by breath variability in $V_T$. This variability tends to be more pronounced in alert patients, and vice versa. In patients with considerable breath by breath variability, inspiratory hold manoeuvres applied randomly produce data at different volumes but there will be greater concentration of data near average $V_T$. The distribution of data points can be made more even through some intelligent paradigms. For example, if there are plenty of data in the average $V_T$ range, breaths with projected low and high $V_T$ may become preferentially targeted for the inspiratory hold manoeuvre. A variety of intelligent algorithms can be entertained which would assist in anticipating a relatively small or relatively large $V_T$ prior to termination of the inflation phase.

In patients who do not display much $V_T$ variability, the data, according to the random routine, fall in a limited volume range. The range can be expanded by reducing or increasing the level of PAV ventilation assist during breaths targeted for the inspiratory hold manoeuvre. Because the change in assist can be perceived by the patient long before the inspiratory hold occurs, and this may result in alterations in $P_{mus}$ during the hold, the reliability of this approach is inversely related to patient alertness. Since the absence of important $V_T$ variability is usually observed in less alert patients, there is some security in applying this procedure in patients with small $V_T$ variability. Further assurance can be obtained by asking the user (attendant) to input a rating of patient's level of alertness with the procedure being implemented only in patients deemed to have low vigilance.

In cases where $P_{aw}$ during the hold approaches but does not reach an asymptote before the minimum latency for behavioral responses (e.g. Figure 2, Graph A), it may be useful to measure $P_{aw}$ at different points between 0 and minimum latency (e.g. 0 to about 300 msec of the inspiratory hold) and use a non-linear function to estimate the asymptote value. Such extrapolation, however, should preferably not be extended beyond an additional about 200 msec and be applied only when the $P_{aw}$ pattern during the first about 300 msec shows a clear tendency toward an asymptote (e.g. Fig 2).

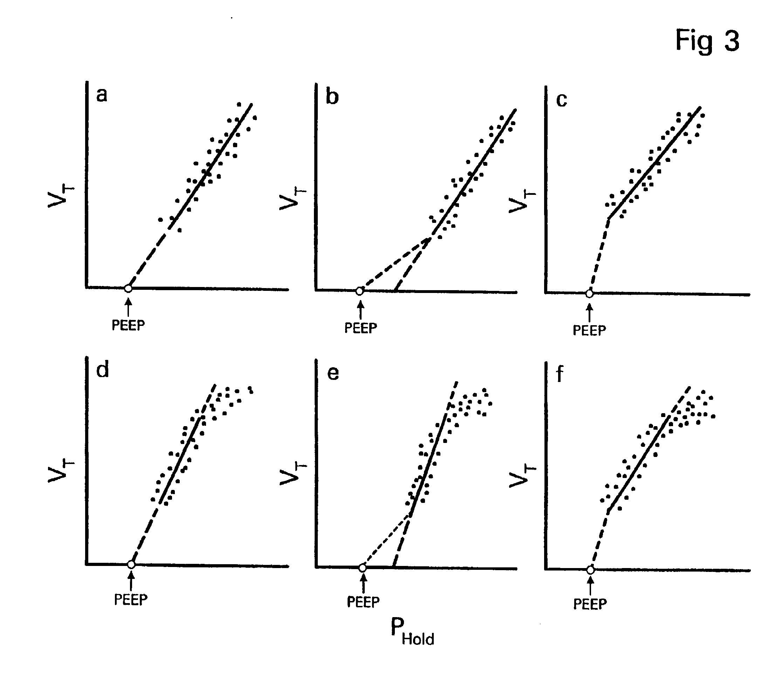

After collection of a suitable number of observations, old data points may be deleted as new ones are acquired, thereby resulting in a continuously updated pressure-volume relation. The results may be displayed in a graphic and/or numerical form. Some examples of graphic patterns are shown in Figure 3. The entire data set might fit a linear function (Figure 3, Graphs a, b, c). Conversely, the data may show relative stiffening in the high $V_T$ range while the linear range in lower $V_T$ range may extrapolate to PEEP or to a pressure above or below PEEP (Figure 3, Graphs d, e, f). Other patterns are also possible. Such displays, and their mathematical counterparts, allow a comprehensive assessment of the pressure-volume relation that is continuously updated.

Although in most patients tested by the procedure of the present invention, the scatter of data points about the regression line is minimal and acceptable, there is, in some patients, substantial scatter reducing the confidence in the slope and intercept of the relation. The inventor has found that, when this occurs, it is often related to ventilatory variables preceding the breath selected for hold and these act to change the volume at the beginning of the selected breath. In other cases, the scatter is related to differences in inspiratory time of the breaths selected for the inspiratory hold. This occurs particularly in patients with pronounced viscoelastic behaviour. When scatter is excessive, ventilatory variables in breaths preceding the selected breaths (e.g. tidal volume, expiratory time, ventilation..etc) as well as inspiratory time of the occluded breaths are incorporated in the regression equation as co-variables. Such procedure invariably reduces the scatter and provides more confident estimates of elastic recoil at different points during inspiration.

Another cause of excessive scatter is the inclusion of faulty data. Such faulty data may occur, for example, when a cough or other erratic development occurs in the course of, or immediately before, the selected breaths. These faulty points may be identified, and deleted, by use of specified criteria including, but not limited to, patterns of flow, $P_{aw}$ and/or volume during or before the selected breaths being clearly outside the normal range of variability established in the particular patient being ventilated.

How to interpret the Results and to set the Volume Assist in the PAV ventilation mode:

i) The average $P_{hold}$ less PEEP is divided by the average $V_T$ to provide a single elastance value (or its reciprocal, compliance). VA can be set to the desired fraction of the single value. This would be clearly appropriate when the P-V relation is linear and with an intercept similar to PEEP (e.g. pattern "a", Figure 3). This approach is not ideal where intercepts exist.

ii) The presence of a $P_{aw}$ intercept that is higher than PEEP (e.g. patterns b and e, Figure 3) signifies either the presence of dynamic hyperinflation (DH) or a stiff respiratory system in the low volume range (e.g. abdominal distension, very small FRC, airway closure...etc). These two possibilities cannot be easily distinguished. Two approaches may be used to provide VA in this case:

a)

$$VA = I + f \bullet V/S$$

where I is the difference between pressure intercept and PEEP, S is the slope of the linear part of the PV curve, V is instantaneous volume, and f is the fraction of elastic load to be unloaded.

b) The lowest data range is connected to PEEP (dashed lines, patterns b and e, Figure 3) and VA becomes a fraction (set by the user) of the pressure-volume relation defined by the dashed extrapolated segment and the regression line obtained from actual data. Where the actual data show stiffening in the upper $V_T$ range, it is desirable to extend the linear function upwards (graph e, Figure 3), although non-linear functions that accommodate the upper stiffening may also be used.

iii) The presence of an intercept that is lower than PEEP signifies either that the breaths begin from a volume below passive FRC or that the entire data set is obtained in the stiff upper range of the P-V curve. In either case, it would be reasonable to connect the lowest actual data points to PEEP (dashed lines, graphs c and f, Figure 3) and VA becomes a desired fraction of the relation defined by the dashed extrapolated line and the linear function obtained from the actual data.

iv) In all cases, suitable non-linear functions may be used.

2) Procedure for Automatic Determination of the Passive Pressure-Flow Relationship (Resistive Properties) (second aspect of invention):

Assessment of the resistive properties of the respiratory system in patients with spontaneous respiratory efforts on mechanical ventilation has been problematic and no satisfactory method currently exists. It is possible to estimate lung resistance using esophageal pressure measurement according to standard methods used in non-ventilated patients. This approach, however, is invasive and the results do not include chest wall resistance. It is also difficult to assess non-linearities in the pressure-flow relation using this technique, particularly in the pressure-support (PSV) and proportional assist (PAV) ventilation modes. For ventilation adjustment of the flow-assist gain of PAV ventilation, it is necessary to consider total respiratory resistance (i.e. not only lung resistance) and non-linearities should preferably also be taken into account.

The inspiratory hold technique, initially introduced for patients on controlled ventilation, has been used also in the assist modes and is currently the most commonly employed approach. This approach, however, is only possible when the patient is in the volume-cycled modes and cannot be applied in PSV or PAV. Furthermore, even in the volume-cycled mode there are problems related to time delays between peak flow and the point of zero flow, which can be as long as about 250 msec. Although in controlled ventilation, this delay can be corrected for, correction is not possible during assisted volume-cycled ventilation since during the delay period patient's effort ($P_{mus}$) could easily have changed, thereby invalidating the basic assumption of the method and producing erroneous results.

Oscillation techniques have been used extensively to measure resistance in non-ventilated humans and in ventilated paralyzed animals. Application of these techniques to mechanically ventilated patients in the assist modes has been hampered by numerous technical problems including non-linear behaviour of the pressure-flow and pressure-volume relations, varying background flow, volume and $P_{mus}$, which necessitate the use of high frequency oscillation so that the slower background changes can be filtered out (however resistance at high frequency is different from resistance at normal breathing frequency), the need to apply the oscillations for a relatively long time, with associated perception problems, which further complicates the analysis.

The present invention, according to this second aspect of the invention, circumvents the above problems using innovative procedures. Furthermore, it is non-invasive, that is not requiring any additional instrumentation of the patient, provides information about the entire pressure-flow relationship in the operating range, including any non-linearities that may exist, and can be easily automated. Although the procedure is suitable for all forms of mechanical ventilation, it is primarily intended for use with PAV ventilation in order to provide a continuously updated estimate of the resistive pressure-flow relationship that can be used to adjust the flow assist component of PAV ventilation.

The features of the method of the present invention according to this second aspect of the invention, therefore, comprise the following:

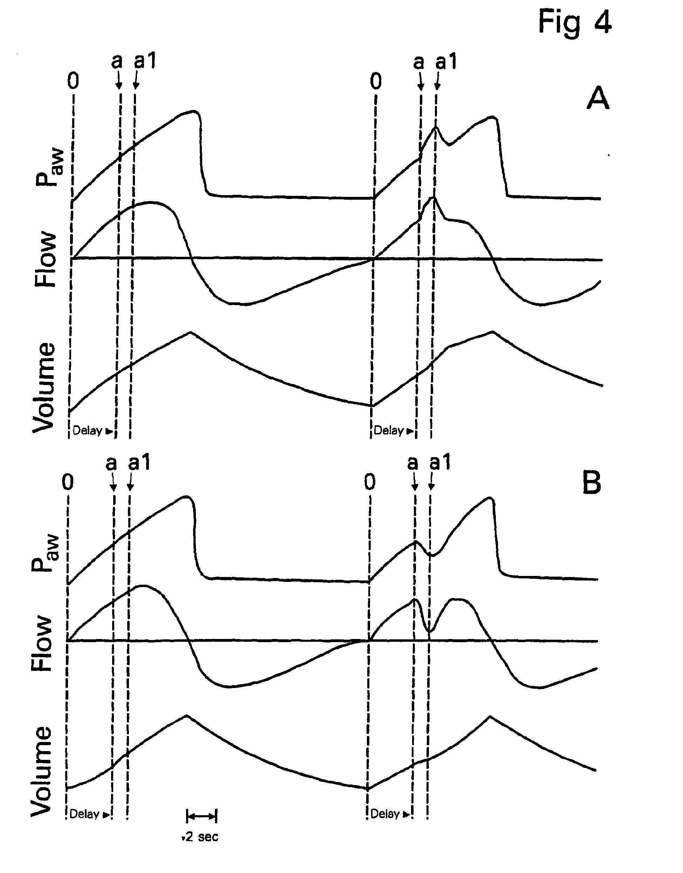

a) applying brief mechanical perturbations in the course of ongoing inspiratory phases, said mechanical perturbation beginning at a specified time or flow after the onset of inspiratory phase with only one perturbation being applied in a given breath (e.g. see Figure 4);

b) limiting the analysis of the consequences of these perturbations to a period that is less than the minimum latency for behavioural responses (preferably $\leq$ about 250 msec);

c) applying perturbations of at least two different amplitudes and/or polarity (i.e. different forms) with the amplitudes being selected to result in a flow range during the perturbations that spans the flow range encountered in unperturbed breaths. Thus, if perturbations are to be applied at a time when flow is naturally high, one may select negative perturbations of two or more amplitudes, with the amplitude of the largest negative perturbation being selected to reduce flow to near zero (e.g. Figure 4B). Alternatively, if the perturbations are to be given at a point where flow is naturally close to zero, two or three positive perturbations may be used with the largest resulting in a flow that approximates the maximum flow observed in unperturbed breaths. If the perturbations are to be applied at a point where flow is intermediate, then a mix of positive and negative perturbations can be used to obtain the desired flow range;

d) measuring pressure ($P_{aw}$), flow ($\dot{V}$) and volume (V) at suitable intervals during the perturbation and storing the results;

e) averaging the results from multiple determinations with each type/amplitude of perturbation in order to eliminate the effect of random breath by breath variability in background conditions (for example, due to differences in $P_{mus}$ or volume at beginning of inspiration ...etc) at the selected time of measurement. When a suitable number of observations per perturbation form is obtained, differences between average pressure, flow and volume among different perturbation forms can be reliably attributed to the perturbations themselves and not to different background conditions.

f) Pressure, flow and volume in unperturbed breaths may also be measured over an identical or similar time interval as the time of measurements in perturbed breaths (Figure 4) and a suitable number of such observations is also averaged providing an additional set of data to define the non-linear pressure-flow relation. A minimum of two types of perturbations and the data from unperturbed breaths are needed to define the non-linear behaviour. Alternatively, data from unperturbed breaths can be replaced by one additional kind of perturbation. Thus, one may use positive perturbations of two different amplitudes and a negative perturbation or two different negative perturbations and one positive perturbation. Other permutations are clearly possible, the main intended result is to produce at least three sets of averaged data (for $P_{aw}$, $\dot{V}$ and V) in which flow covers a wide range.

g) Differences in average $P_{aw}$ between the different sets.are related to the different flow and different volumes. Although these sets of results can be solved with standard mathematical methods to produce values for resistance and elastance, it is preferable to obtain values of elastance independently and allow for the impact of differences in volume on $P_{aw}$ in the data sets using these independently determined elastance values. This is because, in view of the brief nature of the perturbation and relatively small changes in flow, the differences in volume among different data sets in typically very small (e.g. Figure 4). Elastances calculated from the data sets collected in accordance with the present invention may be erroneous in the presence of even minor noise.

Because the procedures for measuring elastic (procedure 1 above) and resistive properties (procedure 2 above) are intended to run concurrently, the elastance values obtained from procedure 1 can be used to estimate the elastic pressure associated with the difference in volume among data sets and, hence, to arrive at the pressure related to difference in flow ($P_{res}$). Thus,

$$\triangle P_{res} = \triangle P_{aw} - \triangle V \bullet E$$

Where $\triangle P_{aw}$ is the difference in average $P_{aw}$ between two sets of observations, $\triangle V$ is the difference in volume between the two sets and E is the incremental elastance determined from procedure 1 or other suitable method. The value of $\triangle P_{res}$ so determined is attributed to the difference in flow between the two sets. In the presence of three or more sets spanning different flow ranges, it is possible, using standard mathematical techniques, to estimate with precision the pressure-flow relation over the entire relevant flow range, including any non-linearities.

The specific features of the procedure according to this aspect of the present invention which overcome prior art problems include:

a) Application of perturbations at a specific time into the inspiratory phase. This feature, after averaging of a number of observations, ensures that differences between variables measured during the perturbation are related to the perturbation and not to varying background conditions.

b) Limitation of analysis to a time within the latency for behavioural responses. This feature eliminates the confounding influence of these responses.

c) Because only one perturbation is applied in a given breath, and there is a time window of about 200 to 300 msec to apply it, the perturbation frequency content can be sufficiently low so that frequency dependence of resistance does not seriously affect results.

d) Because only one perturbation is made in a given breath and perturbations are applied infrequently, the amplitude of the flow perturbation can be relatively large. This feature is not feasible with conventional oscillation techniques where repetitive oscillations over relatively long periods are produced. The relatively large amplitude of flow perturbations permits the determination of the pressure-flow relationship over the entire relevant range while causing little or no discomfort to the patient.

Thus, the present invention offers all the advantages of the conventional oscillation techniques while eliminating the difficulties associated with its implementation in this clinical setting and while remaining non-invasive, easily automated and permitting determination of non-linear behaviour over a wide flow range.

Preferred Procedure for Pressure-Flow Relationship:

Inspiratory flow to the patient $(\dot{V})$ and its integral, volume (V), along with airway pressure ($P_{aw}$) are continuously measured using standard techniques described in the prior art. These measurements can be effected with instrumentation resident in the ventilator or may be included in an external freestanding device. The signals are fed into a computer. Apart from its data acquisition capabilities, the computer is capable of sending out electric command signals (pulses) that are capable of altering $P_{aw}$ and/or $\dot{V}$ either by interfacing directly with the ventilator control system or by altering the output of an external mechanical system attached to the ventilator tubing.

Through suitable analytic algorithms, the computer determines the average time, from onset of inspiration, at which flow is approximately in the midrange of its natural fluctuation. There follows a series of positive and negative perturbations of different amplitudes the intent of which is to determine the amplitude of positive output "pulses" required to increase flow to nearly the peak level usually reached in unperturbed breaths and amplitude of negative output "pulses" required to reduce flow to near zero when applied at the selected time. No more than one "pulse" is given per breath and, preferably, several breaths should separate any two perturbations.

Data collection begins once the time of delivery and amplitude of pulses is decided upon. At intervals, preferably random to avoid anticipatory responses in alert patients, the computer sends out a pulse (positive or negative in random or predetermined sequence) at the selected "delay" from the onset of inspiration ("delay" , Figure 4). $P_{aw}$, $\dot{V}$, V values are sampled immediately before the perturbation (e.g. lines a, Figure 4) and at appropriate intervals during the period of the perturbation (e.g. every about 5 or about 10 msec for about 200 to 300 msec). Sampling is done at the same times in all types of perturbations and, also at the same times from onset of inspiration, in some unpertubated breaths (Figure 4, left breaths). The different values are stored, being segregated according to variable ($P_{aw}$, $\dot{V}$, V) and perturbation type.

As new perturbations are made, more data are added to the tables and average values of $P_{aw}$, $\overset{\bullet}{V}$, and V are computed for all three conditions (unperturbed, positive perturbation, negative perturbation). The data points obtained immediately before the selected perturbation time in perturbed and unperturbed breaths (lines "a", Figure 4) can be used to establish when an adequate number of observations has been collected. This would be when the average values for these initial data (lines "a", Figure 4) obtained in perturbed and unperturbed breaths are very similar, thereby indicating that random breath by breath variability has been addressed by the number of samples gathered and the three sets of data started with similar conditions.

At this point there are three sets of average values related to positive perturbations (+), negative perturbations (-) and unperturbed (u) breaths. A variety of mathematical approaches can be used to extract the pressure-flow relationship from these averaged results. One simple approach is to define a single time during the perturbations that is at or close to peak flow in positive perturbations and at or close to nadir of flow in negative perturbations (e.g. lines "al", Fig. 4). $P_{aw}$, $\overset{\bullet}{V}$ and V data at this time, obtained from positive and negative perturbations and from unperturbed breaths, are then used to compute two incremental resistances, IR (+) and IR (-),

$$ IR(+) \; = \; [P_{aw}(+) \; - \; P_{aw}(u) \; - \; \blacktriangle V(+) \; \times \; E] \; / \; [\overset{\bullet}{V}(+) \; - \; \overset{\bullet}{V}(u)] $$

where $P_{aw}$ (+) is the average $P_{aw}$ during the positive perturbation (as in line "al", Figure 4), $P_{aw}(u)$ is average $P_{aw}$ at the same time in unperturbed breaths, ▲V is the difference in average volume between breaths with positive pulses at time "al" (Figure 4) and unperturbed breaths at the same time, E is incremental elastance determined from the concurrently operating procedure for determining elastance (procedure 1), $\overset{\bullet}{V}(+)$ is average flow at time "al" in breaths with positive perturbations and $\overset{\bullet}{V}(u)$ is average flow in unperturbed breaths at the same time. IR(+) is incremental resistance between $\overset{\bullet}{V}(u)$ and $\overset{\bullet}{V}(+)$.

Likewise, incremental resistance for negative pulses (IR(-)) can be computed:

$$ IR(-) \; = \; [P_{aw}(u) \; - \; P_{aw}(-) \; - \; \blacktriangle V(-) \; \times \; E] \; / \; [\overset{\bullet}{V}(u) \; - \; \overset{\bullet}{V}(-)] $$

where $P_{aw}$ (-) and $\overset{\bullet}{V}(-)$ are average $P_{aw}$ and $\overset{\bullet}{V}$, respectively, at time "al" (Figure 4) in breaths with negative perturbations and ▲V(-) is the difference in average volume at time "a1" between unperturbed breaths and breaths with negative perturbations.

From IR(+) and IR(-), the constants $K_1$ and $K_2$ in Rohrer's equation $(P_{res} \; = \; K_1 \; \bullet \; \overset{\bullet}{V} \; + \; K_2 \; \times \; \overset{\bullet}{V}{}^2)$ can be computed. Thus,

$$ K_2 \; = \; [IR(+) \; - \; IR(-)] \; / \; [\overset{\bullet}{V}(+) \; - \; \overset{\bullet}{V}(-)] $$

where $K_2$ is the constant related to turbulent flow, and

$$ K_1 \; = \; IR(+) \; - \; K_2 \; [\overset{\bullet}{V}(+) \; + \; \overset{\bullet}{V}(u)] $$

or,

$$K_1 = IR(-) - K_2 [\dot{V}(-) + \dot{V}(u)]$$

where $K_1$ is the constant related to laminar flow.

In practice, after a suitable number of observations is collected and $K_1$ and $K_2$ are computed, the frequency of application of perturbations can be reduced. Old data can be deleted from the tables as new ones are added, so that the pressure-flow relation is continuously updated. Values for $K_1$ and $K_2$, obtained at different times during mechanical ventilation can be stored so as to allow the display of time-dependent trends in the pressure-flow relation.

Modifications to the above preferred procedure for establishing the pressure-flow relationship are possible. These include, but are not limited to:

a) Time of application of perturbation need not be in the flow midrange but can be at any time during inspiration. The type and amplitude of perturbations would have to be adjusted accordingly.

b) Perturbations may be applied at more than one selected time. In this case, however, data must be collected from unperturbed breaths or from other perturbations at each of the times selected for application of perturbations.

c) Clearly more than two types of perturbations can be applied. This would increase confidence in the values of $K_1$ and $K_2$ but would consume more time to arrive at a satisfactory solution.

d) Pertubations may be biphasic within the latency for behavioural responses. Likewise more than one biphasic perturbation can be included within this latency period. Although this may reduce the number of breaths that need to be perturbed to arrive at the pressure-flow relationship, the results may be affected by frequency dependence of resistance.

e) Data from unperturbed breaths may be omitted. In such case, however, it is necessary to have a minimum of three types of perturbation to establish the extent of non-linearity in the pressure flow relation.

f) Computation of IR values may be carried out before the baseline (initial) values (measured at lines "a", Figure 4) had equalized in the different data sets. In such case the average values during perturbations need to be adjusted to reflect differences in initial conditions.

g) The actual method of causing a perturbation may vary. Some examples may include adding a transient positive or negative input to the basic control signal of the ventilator. Alternatively, particularly in the PAV mode of ventilation, perturbations can be produced by transient changes in the gain of the flow assist and/or volume assist. Perturbations can also be produced by mechanical devices that are independent of the main ventilator and which are connected to the tubing.

h) Individual breath data may be used to establish the pressure-flow relationship, in lieu of averaged data.- This may be expected, however, to include considerable noise with less confidence in the resulting statistical function.

i) Other mathematical methods of data analysis may be used to arrive at the values of $K_1$ and $K_2$ in Rohrer's equation. Likewise, the data collected from procedures described in this application may be fitted to mathematical functions other than Rohrer's equation.

j) It is not entirely necessary to sample and store data from multiple points during each perturbation. Estimates of the pressure-flow relationship can be obtained if sampling is limited to one or two points in time during each perturbation provided that sampling time, relative to onset of perturbation, is approximately the same for all types of perturbation.

With both procedures (1 and 2 above) for the establishment of the passive pressure-volume relationship and of the passive pressure-flow relationship respectively, additional modifications (algorithms) can be added to identify and delete faulty data points from the collected data prior to computation of incremental resistances or of the pressure-volume relationship. Such faulty points may occur due to misapplied perturbations, occurrence of cough or other erratic developments during or before the breath. Such faulty nature may be established according to specified criteria including, but not limited to, patterns of $P_{aw}$, $\dot{V}$, and/or V during breaths used in analysis that are clearly outside the usual range of variability established from the majority of breaths in the patient being ventilated.

It may be desirable to store the parameters of the pressure-flow and pressure-volume relationship, obtained at different times in the course of mechanical ventilation, so that trends over time in these relations can be reviewed.

## SUMMARY OF DISCLOSURE

In summary of this disclosure, the present invention provides a procedure for determining respiratory mechanics, including passive elastic and resistive properties, during assisted ventilation to provide improved patient ventilation. Modifications are possible within the scope of this invention.

### Claims

1.  A method for estimating a passive pressure-volume relationship of a respiratory system in a patient on mechanical ventilatory support and producing spontaneous respiratory effort (assist mode of ventilation) to guide selection of a volume-assist component of the proportional assist ventilation mode (PAV), which comprises:

    a) placing a ventilator in the proportional assist mode of ventilation, using empiric values of elastance and resistance, or values of elastance and resistance determined by other conventional methods, for initial adjustment of the volume-related and flow-related assist components of the proportional assist ventilation,
    b) monitoring airway pressure ($P_{aw}$) and flow (V) and volume (V) to the patient,
    c) holding flow at or near zero in selected breaths for a period beyond termination of an inspiratory phase of the ventilation,
    d) measuring $P_{aw}$ at a point as far away as possible from the onset of the inspiratory hold but sooner than the latency for behavioral respiratory responses to provide $P_{hold}$.
    e) measuring the tidal volume ($V_T$) of the breaths selected for the inspiratory hold step,

    establishing the relationship between $P_{hold}$ and $V_T$ in said selected breaths to provide a pressure-volume relationship over the $V_T$ range encountered during proportional assist ventilation to permit subsequent adjustment of the volume-related assist for proportional assist ventilation.

2.  The method of claim 1, wherein $P_{hold}$ is measured at a time no greater than about 250 msec from the onset of inspiratory hold.

3.  The method of claim 2, wherein said time is about 200 to about 250 msec from the onset of inspiratory hold.

4.  The method of claim 1, wherein linear functions are employed to fit the relationship between $V_T$ and $P_{hold}$.

5.  The method of claim 1, wherein non-linear functions are used to fit the relation between $V_T$ and $P_{hold}$.

6.  The method of claim 1, wherein $P_{hold}$ is determined by non-linear extrapolation to an asymptote of multiple $P_{aw}$ values measured in the interval between onset of inspiratory hold and latency for behavioral responses.

7.  The method of any one of claims 1 to 6, wherein scatter around a linear or non-linear fit between $V_T$ and $P_{hold}$ is minimized by including factors that can theoretically alter this relation in multiple regression analysis of said relation.

8.  The method of claim 7 wherein said factors are selected from ventilation, $V_T$, expiratory time, expiratory flow and expiratory $P_{aw}$ in the interval preceding the breaths selected for inspiratory hold and/or inspiratory time of the selected breaths.

9.  The method claimed in any one of claims 1 to 8, wherein the gain of volume-assist in PAV is transiently increased or decreased in said selected breaths to cause transient increase or decrease in $V_T$ to expand the range of $V_T$ over which the relation between $V_T$ and $P_{hold}$ is determined.

10. The method claimed in any one of claims 1 to 8, wherein breaths in which $V_T$ is predicted to be substantially larger or smaller than average $V_T$ are preferentially targeted for the inspiratory hold procedure in order to expand the range of $V_T$ over which the relation between $V_T$ and $P_{hold}$ is determined.

11. The method of any one of claims 1 to 10, including eliminating faulty data points from the analysis of the relation between $V_T$ and $P_{hold}$ or between pressure and flow, such faulty nature being established according to specified criteria including patterns of $P_{aw}$, V and/or V during breaths used in analysis, or during breaths preceding said breaths, that are outside the usual range of variability established from the majority of breaths in the patient being ventilated.

12. The method of any one of claims 1 to 11, wherein the pressure-volume and pressure-flow relationships are extrapolated beyond the range of actual observations.

13. The method of any one of claims 1 to 12, wherein old data points are discarded as new ones are collected in order to provide a continuous update of the pressure-volume and pressure-flow relationships.

14. The method of any one of claims 1 to 13, wherein the pressure-volume and pressure-flow relationships computed at different times in the course of mechanical ventilation of a given patient are stored and made available for later display to provide time-related trends in such relationships.

15. The method claimed of any one of claims 1 to 14, wherein the procedures are automated.

16. Apparatus for estimating a passive pressure-volume relationship of a respiratory system in a patient on mechanical ventilatory support and producing spontaneous respiratory effort (assist mode of ventilation) to guide selection of a volume-assist component of the proportional assist ventilation mode (PAV), which comprises:

a) means for placing a ventilator in the proportional assist mode of ventilation, using empiric values of elastance and resistance, or values of elastance and resistance determined by other conventional methods, for initial adjustment of the volume-related and flow-related assist components of the proportional assist ventilation,
b) means for monitoring airway pressure ($P_{aw}$) and flow (V) and volume (V) to the patient,
c) means for holding flow at or near zero in selected breaths for a period beyond termination of an inspiratory phase of the ventilation,
d) means for measuring $P_{aw}$ at a point as far away as possible from the onset of the inspiratory hold but sooner than the latency for behavioral respiratory responses to provide $P_{hold}$,
e) means for measuring the tidal volume ($V_T$) of the breaths selected for the inspiratory hold step,
f) means for establishing the relationship between $P_{hold}$ and $V_T$ in said selected breaths to provide a pressure-volume relationship over the $V_T$ range encountered during proportional assist ventilation to permit subsequent adjustment of the volume-related assist for proportional assist ventilation.

17. The apparatus of claim 16, including means for effecting non-linear extrapolation to an asymptote of multiple $P_{aw}$ values measured in the interval between onset of inspiratory hold and latency for behavioral responses to determined $P_{hold}$.

18. The apparatus of claim 16 or 17, including means for minimizing scatter around a linear or non-linear fit between $V_T$ and $P_{hold}$ by including factors that can theoretically alter this relation in multiple regression analysis of said relation.

19. The apparatus of claim 18 wherein said factors are selected from ventilation, $V_T$, expiratory time, expiratory flow and expiratory $P_{aw}$ in the interval preceding the breaths selected for inspiratory hold and/or inspiratory time of the selected breaths.

20. The apparatus of any one of claims 16 to 19, including means for transiently increasing or decreasing the gain of volume-assist in PAV in the selected breaths to cause transient increase or decrease in $V_T$ to expand the range of $V_T$ over which the relation between $V_T$ and $P_{hold}$ is determined.

21. The apparatus of any one of claims 16 to 20, including means for eliminating faulty data points from the analysis of the relation between $V_T$ and $P_{hold}$ or between pressure and flow, such faulty nature being established according to specified criteria including patterns of $P_{aw}$, V and/or V during breaths used in analysis, or during breaths preceding said breaths, that are outside the usual range of variability established from the majority of breaths in the patient being ventilated.

22. The apparatus of any one of claims 16 to 21, including means for extrapolating the pressure-volume and pressure-flow relationships beyond the range of actual observations.

23. The apparatus of any one of claims 16 to 22, including means for discarding old data points as new ones are collected in order to provide a continuous update of the pressure-volume and pressure-flow relationships.

24. The apparatus of any one of claims 16 to 23, including means for storing the pressure-volume and pressure-flow relationships computed at different times in the course of mechanical ventilation of a given patient, and means for displaying the stored values to provide time-related trends in such relationships.

25. The apparatus of any one of claims 16 to 24, including means for automatically adjusting the volume-assist and/or flow-assist gains of the proportional assist ventilation based on the estimated values.


**Patentansprüche**

1. Verfahren zum Einschätzen des Passivdruck-Volumen-Verhältnisses eines Atmungssystems bei einem Patienten mit mechanischer Ventilationshilfe und Erzeugen einer spontanen respiratorischen Bemühung (Unterstützungsventilationsmodus), um die Wahl der Komponente der Volumenunterstützung des proportionalen Unterstützungsventilationsmodus (PAV) zu bestimmen, umfassend:

a) Einstellen eines Beatmungsgeräts auf den proportionalen Unterstützungsventilationsmodus mittels empirischer Elastanz- und Widerstandswerte oder Elastanz- und Widerstandswerte, die durch andere herkömmliche Verfahren bestimmt werden, zum anfänglichen Einstellen der volumenbezogenen und strömungsbezogenen Unterstützungskomponenten der proportionalen Unterstützungsventilation,
b) Überwachen des Luftwegdrucks ($P_{aw}$) und der Strömung (v) und des Volumens (V) für den Patienten,
c) Halten der Strömung bei oder nahe bei Null in auswählten Atemzügen für einen Zeitraum über das Beenden einer inspiratorischen Phase der Ventilation hinaus,
d) Messen von $P_{aw}$ an einem Punkt, der so weit wie möglich vom Beginn des inspiratorischen Anhaltens entfernt ist aber früher als die Latenz für respiratorische Verhaltensreaktionen liegt, um $P_{Anhalten}$ vorzusehen,
e) Messen des Atemzugvolumens ($V_T$) der Atemzüge, die für den inspiratorischen Anhalteschritt ausgewählt wurden,

wobei das Verhältnis zwischen $P_{Anhalten}$ und $V_T$ in den ausgewählten Atemzügen so eingestellt wird, dass ein Druck-Volumen-Verhältnis gegenüber dem $V_T$-Bereich vorgesehen wird, der während der proportionalen Unterstützungsventilation angetroffen wird, um ein anschließendes Regulieren der volumenbezogenen Unterstützung für die proportionale Unterstützungsventilation zu ermöglichen.

2. Verfahren nach Anspruch 1, wobei $P_{Anhalten}$ zu einer Zeit von nicht mehr als etwa 250 msec vom Beginn des inspiratorischen Anhaltens gemessen wird.

3. Verfahren nach Anspruch 2, wobei die Zeit etwa 200 bis etwa 250 msec vom Beginn des inspiratorischen Anhaltens beträgt.

4. Verfahren nach Anspruch 1, wobei lineare Funktionen verwendet werden, um das Verhältnis zwischen $V_T$ und $P_{Anhalten}$ anzupassen.

5. Verfahren nach Anspruch 1, wobei nicht lineare Funktionen verwendet werden, um das Verhältnis zwischen $V_T$ und $P_{Anhalten}$ anzupassen.

6. Verfahren nach Anspruch 1, wobei $P_{Anhalten}$ durch eine nicht lineare Extrapolation für eine Asymptote der mehreren $P_{aw}$-Werte bestimmt wird, die im Intervall zwischen dem Beginn des inspiratorischen Anhaltens und der Latenz für Verhaltensreaktionen gemessen werden

7. Verfahren nach einem der Ansprüche 1 bis 6, wobei eine Streuung um eine lineare oder nicht lineare Anpassung zwischen $V_T$ und $P_{Anhalten}$ durch Einschließen von Faktoren minimiert wird, die theoretisch dieses Verhältnis in einer Mehrfachregressionsanalyse dieses Verhältnisses ändern können.

8. Verfahren nach Anspruch 7, wobei die Faktoren aus Ventilation, $V_T$, Expirationszeit, Expirationsströmung und expiratorischem $P_{aw}$ im Intervall ausgewählt werden, das den Atemzügen vorangeht, die für das inspiratorische Anhalten und/oder die Inspirationszeit der ausgewählten Atemzüge ausgewählt werden.

9. Verfahren nach einem der Ansprüche 1 bis 8, wobei der Zuwachs der Volumensunterstützung bei der PAV in den ausgewählten Atemzügen vorübergehend gesteigert oder gesenkt wird, um einen vorübergehenden Anstieg oder Abfall von $V_T$ zu bewirken und so den Bereich von $V_T$, gegenüber dem das Verhältnis zwischen $V_T$ und $P_{Anhalten}$ bestimmt wird, zu erweitern.

10. Verfahren nach einem der Ansprüche 1 bis 8, wobei die Atemzüge, bei denen $V_T$ wie vorhergesagt wesentlich größer oder kleiner als der durchschnittliche $V_T$ ist, vorzugsweise für den Ablauf des inspiratorischen Anhaltens ins Auge gefasst werden, um den Bereich von $V_T$, gegenüber dem das Verhältnis zwischen $V_T$ und $P_{Anhalten}$ bestimmt wird, auszuweiten.

11. Verfahren nach einem der Ansprüche 1 bis 10, umfassend das Ausschließen von fehlerhaften Datenpunkten aus der Analyse des Verhältnisses zwischen $V_T$ und $P_{Annalten}$ oder zwischen dem Druck und der Strömung, wobei eine solche fehlerhafte Eigenschaft gemäß bestimmter Kriterien festgestellt wird, die Muster von $P_{aw}$, v und/oder V während der in der Analyse verwendeten Atemzüge oder während der diesen Atemzügen vorangehenden Atemzüge, die außerhalb des üblichen Bereichs der Variabilität liegen, die durch die Mehrzahl der Atemzüge beim ventilierten Patienten eingestellt werden, umfassen.

12. Verfahren nach einem der Ansprüche 1 bis 11, wobei die Druck-Volumen-und Druck-Strömungs-Verhältnisse außerhalb des Bereichs der eigentlichen Beobachtungen extrapoliert werden.

13. Verfahren nach einem der Ansprüche 1 bis 12, wobei alte Datenpunkte verworfen werden, wenn neue gesammelt werden, um ein kontinuierliches Update der Druck-Volumen- und Druck-Strömungs-Verhältnisse vorzusehen.

14. Verfahren nach einem der Ansprüche 1 bis 13, wobei die Druck-Volumen-und Druck-Strömungs-Verhältnisse, die zu unterschiedlichen Zeiten im Verlauf der mechanischen Ventilation eines bestimmten Patienten berechnet werden, gespeichert werden und für eine spätere Anzeige zum Vorsehen von zeitabhängigen Verläufen bei solchen Verhältnissen zur Verfügung gestellt werden.

15. Verfahren nach einem der Ansprüche 1 bis 14, wobei die Abläufe automatisiert sind.

16. Vorrichtung zum Einschätzen eines Passivdrucks-Volumen-Verhältnisses eines Atmungssystems bei einem Patienten mit mechanischer Ventilationshilfe und Erzeugen einer spontanen respiratorischen Bemühung (Unterstützungsventilationsmodus), um Die Wahl der Komponente der Volumenunterstützung des proportionalen Unterstützungsventilationsmodus (PAV) zu bestimmen, umfassend:

a) Mittel zum Einstellen eines Beatmungsgeräts auf den proportionalen Unterstützungsventilationsmodus mittels empirischer Elastanz- und Widerstandswerte oder Elastanz- und Widerstandswerte, die durch andere herkömmliche Verfahren bestimmt werden, zum anfänglichen Einstellen der volumenbezogenen und strömungsbezogenen Unterstützungskomponenten der proportionalen Unterstützungsventilation,
b) Mittel zum Überwachen des Luftwegdrucks ($P_{aw}$) und der Strömung (v) und des Volumens (V) für den Patienten,
c) Mittel zum Halten der Strömung bei oder nahe bei Null in auswählten Atemzügen für einen Zeitraum über das Beenden einer inspiratorischen Phase der Ventilation hinaus,
d) Mittel zum Messen von $P_{aw}$ an einem Punkt, der so weit wie möglich vom Beginn des inspiratorischen Anhaltens entfernt ist aber früher als die Latenz für respiratorische Verhaltensreaktionen liegt, um $P_{Anhalten}$ vorzusehen,
e) Mittel zum Messen des Atemzugvolumens ($V_T$) der Atemzüge, die für den inspiratorischen Anhalteschritt ausgewählt sind,
f) Mittel zum Einstellen des Verhältnisses zwischen $P_{Anhalten}$ und $V_T$ in den ausgewählten Atemzügen, um ein Druck-Volumen-Verhältnis gegenüber dem $V_T$-Bereich vorzusehen, der während der proportionalen Unterstützungsventilation angetroffen wird, um ein anschließendes Regulieren der volumenbezogenen Unterstützung für die proportionale Unterstützungsventilation zu ermöglichen.

17. Vorrichtung nach Anspruch 16, umfassend Mittel zum Bewirken einer nicht linearen Extrapolation für eine Asymptote von mehreren $P_{aw}$-Werten, die im Intervall zwischen dem Beginn des inspiratorischen Anhaltens und der Latenz für Verhaltensreaktionen auf einen bestimmten $P_{Anhalten}$ gemessen werden.

18. Vorrichtung nach Anspruch 16 oder 17, umfassend Mittel zum Minimieren der Streuung um eine lineare oder nicht lineare Anpassung zwischen $V_T$ und $P_{Anhalten}$ durch Einschließen von Faktoren, die theoretisch dieses Verhältnis in einer Mehrfachregressionsanalyse dieses Verhältnisses ändern können.

**19.** Vorrichtung nach Anspruch 18, wobei die Faktoren aus Ventilation, $V_T$, Expirationszeit, Expirationsströmung und expiratorischem $P_{aw}$ im Intervall ausgewählt werden, das den Atemzügen vorangeht, die für das inspiratorische Halten und/oder die Inspirationszeit der ausgewählten Atemzüge ausgewählt werden.

**20.** Vorrichtung nach einem der Ansprüche 16 bis 19, umfassend Mittel zum vorübergehenden Erhöhen oder Senken des Zuwachses der Volumenunterstützung bei der PAV in den ausgewählten Atemzügen, um einen vorübergehenden Anstieg oder Abfall von $V_T$ zu bewirken und so den Bereich von $V_T$, gegenüber dem das Verhältnis zwischen $V_T$ und $P_{Anhalten}$ bestimmt wird, zu erweitern.

**21.** Vorrichtung nach einem der Ansprüche 16 bis 20, umfassend Mittel zum Ausschalten von fehlerhaften Datenpunkten aus der Analyse des Verhältnisses zwischen $V_T$ und $P_{Anhalten}$ oder zwischen dem Druck und der Strömung, wobei eine solche fehlerhafte Eigenschaft gemäß bestimmter Kriterien festgestellt wird, die Muster von $P_{aw}$, v und/oder V während der in der Analyse verwendeten Atemzüge oder während der diesen Atemzügen vorangehenden Atemzügen, die außerhalb des üblichen Bereichs der Variabilität liegen, die durch die Mehrzahl der Atemzüge beim ventilierten Patienten eingestellt werden, umfassen.

**22.** Vorrichtung nach einem der Ansprüche 16 bis 21, umfassend Mittel zum Extrapolieren der Druck-Volumen- und Druck-Strömungs-Verhältnisse außerhalb des Bereichs der eigentlichen Beobachtungen.

**23.** Vorrichtung nach einem der Ansprüche 16 bis 22, umfassend Mittel zum Verwerfen von alten Datenpunkten, wenn neue gesammelt werden, um ein kontinuierliches Update der Druck-Volumen- und Druck-Strömungs-Verhältnisse vorzusehen.

**24.** Vorrichtung nach einem der Ansprüche 16 bis 23, umfassend Mittel zum Speichern der Druck-Volumen- und Druck-Strömungs-Verhältnisse, die zu unterschiedlichen Zeiten im Verlauf der mechanischen Ventilation eines bestimmten Patienten berechnet werden, und Mittel zum Darstellen der gespeicherten Werte, um bei solchen Verhältnissen zeitabhängige Verläufe vorzusehen.

**25.** Vorrichtung nach einem der Ansprüche 16 bis 24, umfassend Mittel zum automatischen Anpassen der Volumensunterstützungs- und/oder Strömungsunterstützungs-Zuwachsraten der proportionalen Unterstützungsventilation auf der Grundlage der geschätzten Werte.

**Revendications**

**1.** Procédé d'estimation d'une relation pression-volume passive d'un système respiratoire d'un patient soutenu par ventilation artificielle et de production d'un effort respiratoire spontané (mode de ventilation assistée) afin de guider une sélection d'un composant d'assistance du volume du mode de ventilation assistée proportionnelle (VAP), qui comprend les étapes consistant à :

a) classer un ventilateur dans le mode de ventilation assistée proportionnelle, en utilisant des valeurs empiriques d'élastance et de résistance, ou des valeurs d'élastance et de résistance déterminées par d'autres procédés classiques, pour un ajustement initial des composants d'assistance liés au volume et liés au courant d'air de la ventilation assistée proportionnelle,

b) surveiller une pression des voies aériennes ($P_{aw}$) et un courant d'air (v) et un volume (V) du patient,

c) retenir un courant d'air à ou près de zéro au cours de respirations sélectionnées pendant une période au-delà de la fin d'une phase inspiratoire de la ventilation,

d) mesurer $P_{aw}$ au niveau d'un point aussi éloigné que possible du début de la retenue inspiratoire mais avant le temps de latence nécessaire aux réponses respiratoires comportementales afin de fournir $P_{hold}$,

e) mesurer le volume courant ($V_T$) des respirations sélectionnées pour l'étape de retenue inspiratoire,

établir la relation entre $P_{hold}$ et $V_T$ dans lesdites respirations sélectionnées afin de fournir une relation pression-volume sur la plage $V_T$ rencontrée au cours d'une ventilation assistée proportionnelle afin de permettre un ajustement ultérieure de l'assistance liée au volume pour une ventilation assistée proportionnelle.

**2.** Procédé selon la revendication 1, dans lequel $P_{hold}$ est mesuré à un moment qui n'excède pas environ 250 ms depuis le début d'une retenue inspiratoire.

3. Procédé selon la revendication 2, dans lequel ledit moment est à environ 200 à environ 250 ms du début de la retenue inspiratoire.

4. Procédé selon la revendication 1, dans lequel des fonctions linéaires sont utilisées pour adapter la relation entre $V_T$ et $P_{hold}$.

5. Procédé selon la revendication 1, dans lequel des fonctions non linéaires sont utilisées pour adapter la relation entre $V_T$ et $P_{hold}$.

6. Procédé selon la revendication 1, dans lequel $P_{hold}$ est déterminé par une extrapolation non linéaire par rapport à une asymptote de multiples valeurs $P_{aw}$ mesurées dans l'intervalle entre un début d'une retenue inspiratoire et un temps de latence des réponses comportementales.

7. Procédé selon l'une quelconque des revendications 1 à 6, dans lequel une dispersion autour d'une adaptation linéaire ou non linéaire entre $V_T$ et $P_{hold}$ est minimisée en incluant des facteurs qui peuvent théoriquement altérer cette relation dans une analyse de régression multiple de ladite relation.

8. Procédé selon la revendication 7, dans lequel lesdits facteurs sont sélectionnés parmi la ventilation, $V_T$, la durée expiratoire, le courant d'air expiratoire et la $P_{aw}$ expiratoire dans l'intervalle précédant les respirations sélectionnées pour une retenue inspiratoire et/ou une durée inspiratoire des respirations sélectionnées.

9. Procédé selon l'une quelconque des revendications 1 à 8, dans lequel le gain d'une assistance du volume dans une VAP est augmenté ou diminué de manière transitoire dans lesdites respirations sélectionnées afin d'amener une augmentation ou une diminution transitoire dans $V_T$ afin d'étendre la plage de $V_T$ sur laquelle la relation entre $V_T$ et $P_{hold}$ est déterminée.

10. Procédé selon l'une quelconque des revendications 1 à 8, dans lequel des respirations dans lesquelles $V_T$ est prédit comme étant sensiblement plus grand ou plus petit qu'un $V_T$ moyen sont de préférence ciblées pour la procédure de retenue inspiratoire afin d'étendre la plage de $V_T$ sur laquelle la relation entre $V_T$ et $P_{hold}$ est déterminée.

11. Procédé selon l'une quelconque des revendications 1 à 10, comportant une élimination des points de données erronés de l'analyse de la relation entre $V_T$ et $P_{hold}$ ou entre une pression et un courant d'air, une telle nature erronée étant établie selon des critères spécifiés comportant des motifs de $P_{aw}$, (v) et/ou V au cours de respirations utilisées dans une analyse, ou au cours de respirations précédant lesdites respirations qui se trouvent hors de la plage habituelle de variabilité établie à partir de la majorité des respirations d'un patient ventilé.

12. Procédé selon l'une quelconque des revendications 1 à 11, dans lequel les relations pression-volume et pression-courant d'air sont extrapolées au-delà de la plage des observations réelles.

13. Procédé selon l'une quelconque des revendications 1 à 12, dans lequel des points de données anciens sont rejetés lorsque de nouveaux points sont collectés afin de fournir une mise à jour continue des relations pression-volume et pression-courant d'air.

14. Procédé selon l'une quelconque des revendications 1 à 13, dans lequel les relations pression-volume et pression-courant d'air calculées à des moments différents au cours d'une ventilation artificielle d'un patient donné sont mémorisées et rendues disponibles pour un affichage ultérieur afin de fournir des tendances temporelles de telles relations.

15. Procédé selon l'une quelconque des revendications 1 à 14, dans lequel les procédures sont automatisées.

16. Appareil d'estimation d'une relation pression-volume passive d'un système respiratoire d'un patient soutenu par ventilation artificielle et produisant un effort respiratoire spontané (mode de ventilation assistée) afin de guider une sélection d'un composant d'assistance du volume du mode de ventilation assistée proportionnelle (VAP) qui comprend :

a) un moyen destiné à placer un ventilateur dans le mode de ventilation assistée proportionnelle, utilisant des valeurs empiriques et une élastance et une résistance, ou des valeurs d'élastance et de résistance déterminées par d'autres procédés classiques, pour un ajustement initial des composants d'assistance liés au volume et liés au courant d'air de la ventilation assistée proportionnelle,

b) un moyen destiné à surveiller une pression des voies aériennes ($P_{aw}$) et un courant d'air (v) et un volume (V) du patient,

c) un moyen destiné à retenir un courant d'air à ou près de zéro dans des respirations sélectionnées pendant une période au-delà de la fin d'une phase inspiratoire de la ventilation,

d) un moyen destiné à mesurer $P_{aw}$ au niveau d'un point aussi éloigné que possible du début de la retenue inspiratoire mais avant le temps de latence des réponses respiratoires comportementales afin de fournir $P_{hold}$,

e) un moyen destiné à mesurer le volume courant ($V_T$) des respirations sélectionnées pour l'étape de retenue inspiratoire,

f) un moyen destiné à établir la relation entre $P_{hold}$ et $V_T$ dans lesdites respirations sélectionnées afin de fournir une relation pression-volume sur la plage $V_T$ rencontrée au cours d'une ventilation assistée proportionnelle afin de permettre un ajustement ultérieur de l'assistance liée au volume pour une ventilation assistée proportionnelle.

17. Appareil selon la revendication 16, comportant un moyen destiné à effectuer une extrapolation non linéaire par rapport à une asymptote de multiples valeurs $P_{aw}$ mesurées dans l'intervalle entre un début d'une retenue inspiratoire et un temps de latence de réponses comportementales afin de déterminer $P_{hold}$.

18. Appareil selon la revendication 16 ou 17, comportant un moyen destiné à minimiser une dispersion autour d'une adaptation linéaire ou non linéaire entre $V_T$ et $P_{hold}$ en incluant des facteurs qui peuvent théoriquement altérer cette relation dans une analyse de régression multiple de ladite relation.

19. Appareil selon la revendication 18 dans lequel lesdits facteurs sont sélectionnés parmi une ventilation, $V_T$, une durée expiratoire, un courant d'air expiratoire et un $P_{aw}$ expiratoire dans l'intervalle précédant les respirations sélectionnées pour une retenue inspiratoire et/ou une durée inspiratoire des respirations sélectionnées.

20. Appareil selon l'une quelconque des revendications 16 à 19, comportant un moyen destiné à augmenter ou diminuer de manière transitoire le gain d'une assistance de volume dans une VAP dans les respirations sélectionnées afin d'amener une augmentation ou une diminution transitoire dans $V_T$ afin d'étendre la plage de $V_T$ sur laquelle la relation entre $V_T$ et $P_{hold}$ est déterminée.

21. Appareil selon l'une quelconque des revendications 16 à 20 comportant un moyen destiné à éliminer des points de données erronés de l'analyse de la relation entre $V_T$ et $P_{hold}$ ou entre une pression et un courant d'air, une telle nature erronée étant établie selon des critères spécifiés comportant des motifs de $P_{aw}$, v et/ou V au cours de respirations utilisées dans une analyse, ou au cours de respirations précédant lesdites respirations, qui sont hors de la plage habituelle de variabilité établie à partir de la majorité des respirations du patient ventilé.

22. Appareil selon l'une quelconque des revendications 16 à 21, comportant un moyen destiné à extrapoler les relations pression-volume et pression-courant d'air au-delà de la plage d'observations réelles.

23. Appareil selon l'une quelconque des revendications 16 à 22, comportant un moyen destiné à rejeter des points de données anciens lorsque de nouveaux points sont collectés afin de fournir une mise à jour continue des relations pression-volume et pression-courant d'air.

24. Appareil selon l'une quelconque des revendications 16 à 23, comportant un moyen destiné à mémoriser des relations pression-volume et pression-courant d'air calculées à des moments différents au cours d'une ventilation artificielle d'un patient donné, et un moyen destiné à afficher les valeurs mémorisées afin de fournir des tendances temporelles de telles relations.

25. Appareil selon l'une quelconque des revendications 16 à 24 comportant un moyen destiné à ajuster automatiquement les gains d'assistance de volume et/ou d'assistance de courant d'air de la ventilation assistée proportionnelle sur la base des valeurs estimées.

Fig 1

Fig. 2

Fig 3

Fig 4